# FASCICULE DE BREVET EUROPEEN

(11) **EP 0 859 571 B1**
(45) Date de publication et mention de la délivrance du brevet: **23.01.2002**
(21) Numéro de dépôt: 96934239.3
(22) Date de dépôt: 04.11.1996
(51) Int. Cl.: A61B 17/00, A61B 17/22, A61B 17/30, A61F 2/06, A61N 1/05

(54) **DISPOSITIF DE CAPTURE A INTRODUIRE DANS UN CONDUIT D'UN CORPS**
FASSVORRICHTUNG ZUM EINFÜHREN IN EIN KÖRPERGEFÄSS
RETRIEVAL DEVICE FOR INSERTION INTO A BODY LUMEN

(30) Priorité: 07.11.1995 BE 9500920
(43) Date de publication de la demande: 26.08.1998
(73) Titulaire: Dereume, Jean-Pierre, George, Emile, B-1050 Bruxelles (BE)
(72) Inventeur: Dereume, Jean-Pierre, George, Emile, B-1050 Bruxelles (BE)
(74) Mandataire: Claeys, Pierre
(86) Numéro de dépôt international: BE9600117
(87) Numéro de publication internationale: WO9717021

(56) Documents cités:
- EP-A- 0 518 839
- EP-A- 0 701 800
- WO-A-93/15671
- WO-A-94/15549
- WO-A-94/26179
- WO-A-96/26696
- DE-A- 3 542 667
- US-A- 4 174 715
- US-A- 4 471 777
- US-A- 4 611 594
- US-A- 4 655 219
- US-A- 4 997 435
- US-A- 5 053 041
- US-A- 5 098 440

## Description

La présente invention est relative à un dispositif de capture à introduire dans un conduit tubulaire biologique d'un corps humain ou animal, pour capturer un matériel tubulaire situé dans ce conduit, comprenant
une gaine porteuse présentant une première cavité axiale,
des moyens de poussoir logés à l'intérieur de la gaine porteuse et capables de coulisser dans ladite première cavité axiale,
des premiers moyens de commande permettant un déplacement relatif entre les moyens de poussoir et la gaine porteuse, et
des moyens de préhension flexibles qui sont portés par les moyens de poussoir et sont déplacés entre une position à l'extérieur de la gaine porteuse, où ils sont écartés l'un de l'autre, et une position à l'intérieur de la gaine porteuse, où ils sont rapprochés l'un de l'autre, les moyens de préhension présentant une extrémité distale courbée vers l'extérieur.

Depuis un certain temps déjà, il est devenu nécessaire de disposer d'un dispositif de capture permettant l'extraction par voie endoluminale d'endoprothèses situées dans un conduit tubulaire biologique du corps humain ou animal. Par conduit tubulaire biologique il faut entendre toutes les voies du corps humain ou animal, par exemple les vaisseaux sanguins, les canaux lymphatiques, les voies urinaires, les voies biliaires, les voies aériennes comme les bronches, l'oesophage, et des parties analogues. Par matériel tubulaire, il faut entendre tout matériel de forme cylindrique placé à l'intérieur de ces conduits tubulaires biologiques, en particulier pour soutenir, remplacer ou dilater une paroi pathologique. Ce matériel est en général en contact intime avec la paroi interne de ces conduits tubulaires. Il peut s'agir notamment d'endoprothèses, de tuteurs métalliques, recouverts ou non d'un revêtement synthétique, de dilatateurs de canaux et d'éléments analogues.

On connaît déjà des dispositifs du genre cité au début (voir US-A-5098440). Le dispositif prévu dans ce brevet est toutefois conçu avec des boucles de préhension à insérer entre le tuteur à capturer et la paroi du vaisseau dans lequel il se trouve. Dans la pratique, il est toutefois extrêmement difficile de procéder à cette insertion, car les matériels tubulaires introduits dans les vaisseaux sont conçus de façon à exercer une dilatation radiale sur les vaisseaux dans lesquels ils se trouvent. De plus, beaucoup d'endoprothèses sont à l'heure actuelle recouvertes d'un revêtement très favorable à la croissance des cellules. Peu de jours après avoir largué l'endoprothèse, il se produit donc déjà un développement des cellules des parois du vaisseau dans le revêtement de l'endoprothèse. L'insertion de boucles métalliques entre l'endoprothèse et le vaisseau devient à partir de ce moment difficilement concevable, sinon impossible. Un autre dispositif de capture de type semblable et fonctionnant de cette même manière est décrit dans la EP-A-0518839.

On peut citer par ailleurs des dispositifs de capture de la firme COOK PACEMAKER CORPORATION, Etats-Unis d'Amérique, qui sont constitués d'une gaine porteuse et d'un fil coulissant à l'intérieur de celle-ci. L'extrémité distale du fil est prévue à la manière d'un lasso que l'on peut serrer quand l'objet à capturer est pris au piège. Cette même firme commercialise un dispositif à "Deflecting Wire" où le fil coulissant à l'intérieur de la gaine porteuse forme une boucle non fermée à son extrémité d'accrochage, ou encore un dispositif du type "Dotter Basket" dans lequel l'extrémité de capture est formée de 3 ou 4 fils réunis à leur extrémité distale. Lorsque ces fils sortent de la gaine porteuse ils forment une espèce de panier fermé, qui est constitué de fils disposés en hélice (voir dépliant publicitaire de la firme COOK PACEMAKER CORPORATION de 1992; CLYDE W. et al, Cardiac Pacemaker Electrodes : Improved Methods of Extraction, Radiology 1994, 193 : 739-742). Pour des dispositifs semblables, voir aussi US-A-4471777, WO-93/15671, US-A-4611594, DE-A-3542667.

On connaît aussi un dispositif de capture, appelé Boren-McKinney Retriever Set, qui présente un double fil de récupération. Ces fils flexibles sont recourbés vers l'intérieur à leur extrémité distale et ils s'écartent l'un de l'autre dans un plan axial lorsqu'ils se trouvent à l'extérieur de la gaine porteuse (CLYDE W. et al, op. cit.). On connaît aussi de tels dispositifs de capture à plusieurs fils recourbés vers l'intérieur qui sont prévus pour récupérer de petits objets situés dans des cavités de grande dimension, que ce soit par laparoscopie (US-A-4174715) ou par endoscopie (US-A-4655219). Ces dispositifs agissent à la manière de grappins.

Aucun de ces dispositifs de capture ne permet par conséquent de saisir des matériels tubulaires, tels que des endoprothèses mal larguées dans des cavités tubulaires, comme des vaisseaux sanguins, des canaux du système gastro-intestinal ou urinaire, ou d'autres endroits analogues.

En effet il peut arriver, par exemple lors du largage d'une endoprothèse dans un vaisseau, que celle-ci se place imparfaitement, et notamment à un endroit où par exemple elle obture partiellement ou totalement l'embouchure d'un autre vaisseau. Il faut dans ce cas soit déplacer l'endoprothèse, en la saisissant et en la faisant glisser au bon endroit, soit récupérer l'endoprothèse pour la remplacer. Aucun des dispositifs précités n'est capable d'effectuer une telle opération.

On connaît aussi des appareils pour éliminer une structure allongée implantée dans un tissu biologique. Ces appareils sont des systèmes relativement complexes et tout à fait complémentaires de l'objet à récupérer, qu'il s'agisse d'un stimulateur cardiaque ou d'un conducteur cardiaque inséré dans une paroi du coeur, par exemple (voir US-A-4943289, US-A-4988347, US-A-5011482 et US-A-5013310).

On peut aussi citer un dispositif de soutien de vaisseau, pourvu de trois crochets à son extrémité (US-A-5053041). Lors d'une opération chirurgicale, ce dispositif sert à maintenir l'extrémité du vaisseau à greffer ouverte, en particulier pendant l'opération de suture. Il n'est pas prévu pour une introduction par voie endoluminale et pour une capture d'objet.

On connaît enfin des dispositifs implantables dans les vaisseaux sanguins. Ces dispositifs sont par exemple formés de 2 faisceaux de fils métalliques réunis entre eux par une bague à partir de laquelle ils s'étendent radialement en ombrelle, des deux côtés. Ces dispositifs sont largués de manière définitive à un endroit donné pour filtrer le sang et empêcher le passage de caillots de sang vers le coeur. Ils ne sont donc aucunement destinés à une capture d'objet, et encore moins à une récupération d'endoprothèse (voir US-A-3868956).

On résout les problèmes cités ci-dessus, par un dispositif tel que décrit au début dans lequel les moyens de préhension comprennent des éléments d'accrochage qui sont disposés périphériquement sur un poussoir de manière à s'étendre vers l'avant, à l'intérieur de la gaine porteuse, et à s'écarter l'un de l'autre radialement à l'extérieur de la gaine porteuse et qui sont chacun pourvus à leur extrémité distale d'un crochet recourbé radialement vers l'extérieur qui, lorsque les éléments d'accrochage sont situés à l'intérieur du matériel tubulaire et sont amenés dans ladite position à l'extérieur de la gaine porteuse, sont capables d'entrer en position d'accrochage avec une surface interne du matériel tubulaire et qui, lorsque les éléments d'accrochage se trouvent dans la position d'accrochage susdite et sont amenés vers ladite position à l'intérieur de la gaine porteuse, permettent de comprimer radialement le matériel tubulaire. Ce dispositif offre l'avantage que l'opérateur ne doit pas perdre de temps pour orienter convenablement radialement les éléments d'accrochage par rapport à l'objet à saisir. Si l'on veut capturer un objet du type endoprothèse, par exemple vasculaire, les éléments d'accrochage, avantageusement munis chacun d'un crochet recourbé radialement vers l'extérieur, entrent en prise avec la paroi interne de l'endoprothèse. Une traction radiale peut alors être exercée sur celle-ci qui provoque son décollement depuis la paroi vasculaire et qui permet sa récupération.

Suivant une forme de réalisation avantageuse, les éléments d'accrochage sont des fils minces flexibles qui, à une extrémité, sont fixés à une extrémité distale du poussoir et sont élastiquement comprimés par la gaine porteuse lorsqu'ils sont à l'intérieur de celle-ci. Suivant cette forme de réalisation, les fils à l'extérieur de la gaine tendent à retrouver leur état de repos dans lequel ils sont courbés. Cela entraîne leur expansion radiale à l'extérieur de la gaine.

Suivant une forme perfectionnée de l'invention, le dispositif comprend en outre
une gaine externe présentant une deuxième cavité axiale, dans laquelle la gaine porteuse est logée de manière à pouvoir coulisser,
des deuxièmes moyens de commande permettant un déplacement relatif entre la gaine externe et la gaine porteuse,
et des moyens de récupération de l'objet capturé par les éléments d'accrochage, qui sont déplaçables entre une position à l'extérieur de la gaine externe et une position à l'intérieur de la gaine externe. Cette forme de réalisation offre l'avantage de récupérer l'objet capturé par les éléments d'accrochage et de le faire entrer dans une gaine externe, en particulier lorsqu'il s'agit d'un objet du type tuteur ou endoprothèse. De préférence, ces moyens de récupération sont constitués d'un tube flexible, qui est comprimé à l'intérieur de la deuxième cavité axiale de la gaine externe et qui, à l'extérieur de celle-ci, s'évase en étant ouvert vers la cavité du corps humain ou animal.

On a déjà décrit de tels moyens de récupération, par exemple en laparoscopie, pour capter des objets dans un panier en forme d'entonnoir extensible vers l'avant (WO 94/26179). Ces moyens n'ont toutefois pas été conçus pour récupérer du matériel tubulaire à l'intérieur de cavités biologiques tubulaires.

D'autres formes de réalisation avantageuses de l'invention sont indiquées dans les revendications données ci-après.

D'autres détails et particularités de l'invention vont à présent être donnés dans la description ci-après d'exemples de réalisation non limitatifs de l'invention, et cela avec référence aux dessins annexés.

La figure 1 représente une vue en perspective, partiellement brisée, d'une forme de réalisation de dispositif de capture suivant l'invention, avec les éléments d'accrochage à l'extérieur de la gaine porteuse.

La figure 2 représente une vue du dispositif illustré à la figure 1, avec les éléments d'accrochage à l'intérieur de la gaine porteuse.

La figure 3 représente une vue en coupe, suivant la ligne III-III, de la figure 6.

La figure 4 représente une vue schématique des moyens de commande à l'extrémité proximale d'un dispositif de capture suivant l'invention.

La figure 5 représente une vue en coupe axiale d'une forme de réalisation particulière de dispositif de capture suivant l'invention, en position rentrée.

La figure 6 représente le dispositif de la figure 5 en position de capture d'une endoprothèse.

La figure 7 représente le dispositif des figures 5 et 6 en cours de récupération de l'endoprothèse.

Les figures 8 et 9 représentent, dans une vue analogue à la figure 3, des variantes de réalisation des éléments d'accrochage.

La figure 10 représente une variante de cône de récupération, fixé à une gaine de récupération.

Sur les différents dessins, les éléments identiques ou analogues sont désignés par les mêmes références.

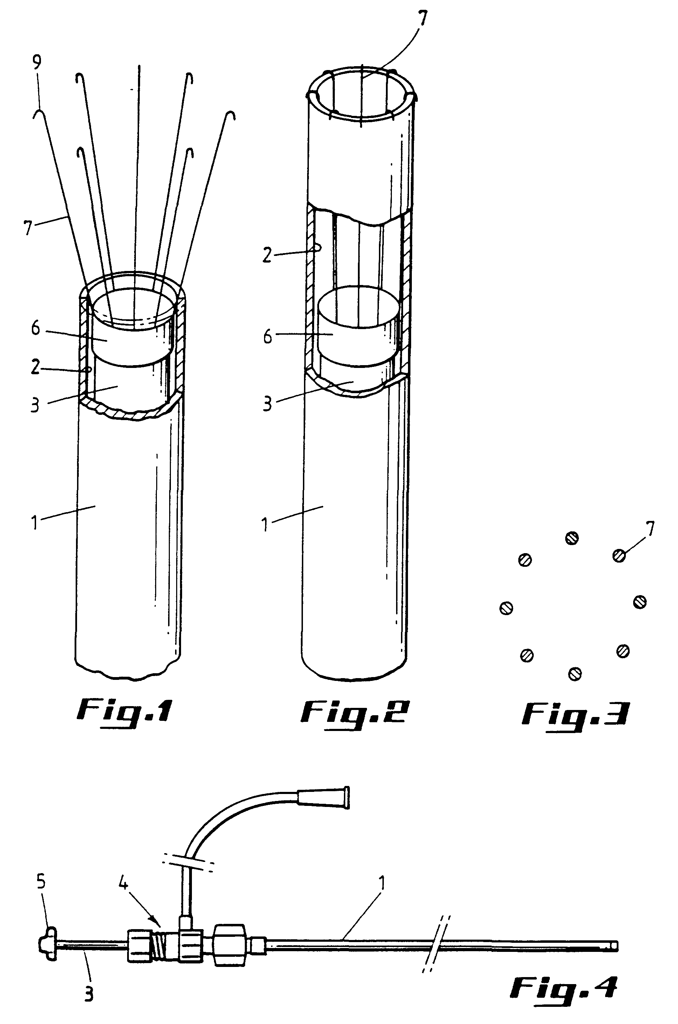

Ainsi qu'il ressort des figures 1 à 4, un dispositif de capture suivant l'invention comprend une gaine porteuse 1 qui présente une cavité axiale 2. Dans celle-ci un poussoir 3 est agencé de manière à pouvoir coulisser.

A l'extrémité proximale du dispositif de capture, représentée sur la figure 4, des moyens de commande usuels sont prévus pour permettre un déplacement relatif entre le poussoir 3 et la gaine porteuse 1. Ces moyens de commande sont par exemple constitués d'une vanne hémostatique 4 qui peut être serrée et desserrée sur le poussoir 3, et d'une poignée 5 pour manipuler le poussoir lorsque la vanne hémostatique 4 est à l'état desserré. De tels moyens de commande sont disponibles dans le commerce, par exemple ceux fabriqués par la firme CORDIS, pour la commande de cathéters et de dispositifs d'introduction d'objets dans les cavités d'un corps humain ou animal. Ces moyens de commande n'ont donc pas besoin d'être décrits de manière plus détaillée.

A l'extrémité distale du poussoir, celui-ci est, dans l'exemple illustré, muni d'une virole 6 qui est sertie à son extrémité. Cette virole permet la fixation d'éléments d'accrochage, illustrés ici en forme de fils flexibles 7, par exemple en acier chirurgical, à l'extrémité du poussoir 3. La fixation des fils 7 sur la virole peut se faire par exemple par soudage ou par tout autre procédé analogue, bien connu de l'homme de métier.

Dans la forme de réalisation illustrée sur la figure 1, les extrémités distales des fils 7 sont recourbées vers l'extérieur en forme de crochets 9. Dans la forme de réalisation illustrée sur les figures 1 à 3, les éléments d'accrochage sont disposés périphériquement sur le poussoir, de manière uniformément espacée, et ils sont agencés au nombre de huit. On pourrait évidemment prévoir une disposition non uniforme et un nombre minimal de trois éléments d'accrochage. Il est toutefois plus efficace d'en prévoir au moins 6, et de préférence au moins 8.

Dans la position représentée sur la figure 2, les fils flexibles 7 sont rapprochés l'un de l'autre à l'intérieur de la gaine porteuse 3. Dans cette position, ces fils en métal ou en une autre matière flexible sont, dans cet exemple, soumis à une contrainte élastique de compression. Dans la position en dehors de la gaine porteuse, illustrée sur la figure 1, les fils flexibles tendent vers leur position de repos et ils s'écartent donc l'un de l'autre, chacun radialement, à la manière des pétales d'une fleur. Ils forment ainsi ensemble un moyen de préhension de forme évasée, qui est ouvert vers l'avant, c'est-à-dire vers la cavité du corps humain ou animal dans laquelle le dispositif de capture est introduit.

On peut aussi prévoir que les éléments d'accrochage 7 soient des fils en un métal spécial à mémoire thermique, par exemple en NITINOL®, qui à la température du corps humain ou animal retrouve sa forme. Dans ce cas, les fils 7 ne sont pas à l'état précontraint à l'intérieur de la gaine porteuse.

La forme de réalisation suivant la figure 1 peut servir à capter un matériel tubulaire du type endoprothèse, tuteur, dilatateur, et éléments analogues, dans des conduits biologiques tubulaires.

On peut par exemple l'utiliser lorsqu'une endoprothèse luminale a été mal larguée. Si, à la suite de cela, elle obture partiellement ou totalement l'embouchure d'une ramification importante d'un vaisseau sanguin par exemple, il faut immédiatement remédier à cette situation.

L'opérateur, après avoir, d'une manière courante pour un homme de métier, introduit le dispositif de capture dans un conduit tubulaire du corps humain, par exemple un vaisseau sanguin, fait parvenir la gaine porteuse 1 contenant le poussoir 3 et les éléments d'accrochage 7 à l'intérieur de sa cavité 2 devant l'objet à capturer, visible sur l'écran de radioscopie. A l'aide des moyens de commande, il peut faire coulisser vers l'avant le poussoir 3 ou tirer vers l'arrière la gaine porteuse 1, de façon à faire sortir les fils 7 de la cavité 2. Les fils 7 s'écartent alors radialement. L'opérateur n'est nullement obligé de faire tourner le poussoir sur son axe pour l'orienter par rapport à l'objet à capturer, il est toujours en position correcte.

L'extrémité distale de la gaine porteuse 1 peut être amenée à l'entrée, ou même à l'intérieur de l'objet tubulaire à capturer. Le poussoir 3 et la gaine porteuse 1 sont alors déplacés, comme indiqué ci-dessus, l'un par rapport à l'autre de façon que les fils d'accrochage 7 s'écartent radialement et que les crochets 9 recourbés vers l'extérieur viennent se planter dans le revêtement interne de l'endoprothèse. En exerçant à ce moment une traction vers l'arrière sur l'ensemble du dispositif de capture, on peut faire coulisser vers l'arrière l'endoprothèse mal larguée. On peut simultanément rentrer légèrement les fils à l'intérieur de la gaine porteuse, ce qui a pour effet d'exercer une traction en sens radial sur l'endoprothèse et de la décoller des parois du vaisseau, avec secondairement un allongement de l'endoprothèse et un glissement plus aisé de celle-ci dans la lumière du vaisseau. Une fois l'endoprothèse placée au bon endroit, on peut repousser très légèrement vers l'avant le poussoir pour décrocher les fils 7 et immédiatement faire coulisser vers l'avant la gaine porteuse 1 pour rapprocher les fils 7 l'un de l'autre et les réintroduire dans la cavité 2. Le dispositif de capture peut alors être extrait du corps de la manière usuelle.

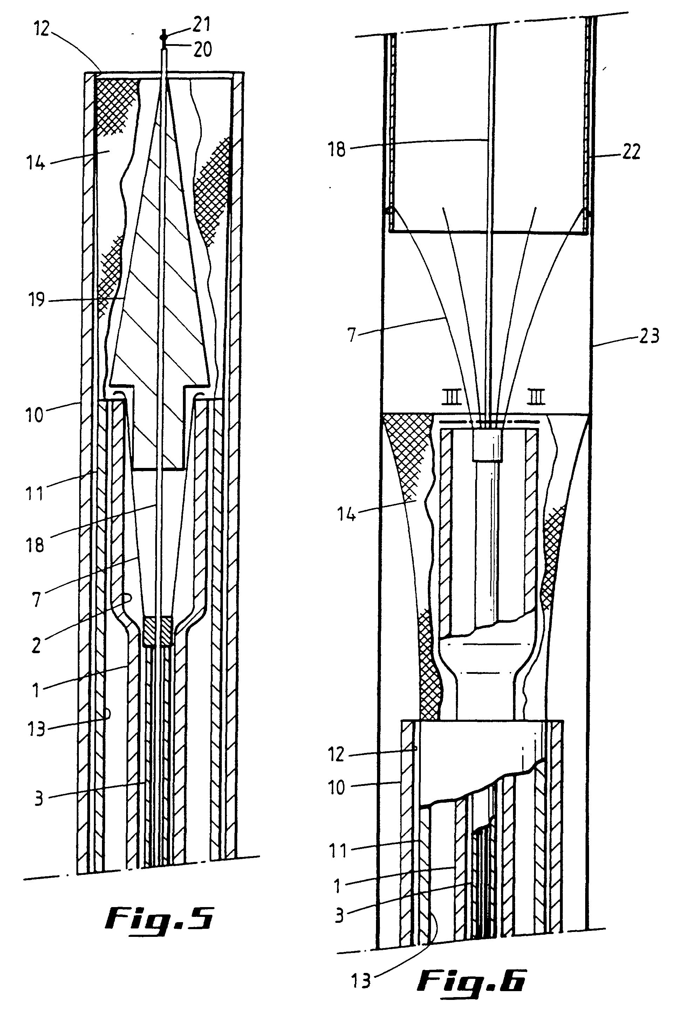

Les figures 5 à 7 représentent une forme de réalisation plus complexe d'un dispositif suivant l'invention.

En plus de la gaine porteuse 1 et du poussoir 3 muni d'éléments d'accrochage 7, on peut prévoir une gaine externe 10 et une gaine de récupération 11. La gaine de récupération 11 est logée dans la cavité axiale 12 de la gaine externe 10 de manière à pouvoir coulisser par rapport à elle. La gaine porteuse 1 est logée dans la cavité axiale 13 de la gaine de récupération 11. Des moyens de commande usuels, tels que des vannes hémostatiques, sont prévus pour le déplacement mutuel de chacune de ces gaines.

Dans l'exemple illustré sur les figures 5 à 7, des moyens de récupération sont agencés à l'extrémité distale de la gaine de récupération 11. Ces moyens de récupération peuvent être constitués d'un tube flexible, qui peut être comprimé élastiquement à l'intérieur de la cavité axiale 12 de la gaine externe 10 et qui, à l'extérieur de celle-ci, s'évase en s'ouvrant vers la cavité du corps humain ou animal. Dans l'exemple illustré, ce tube flexible est formé d'un tuteur auto-expansible 14, qui est radialement expansible et axialement rétractable et qui comprend des premiers fils rigides flexibles, fixés à une extrémité de la gaine de récupération 11 et enroulés suivant un premier sens autour d'un axe longitudinal de celle-ci, et des deuxièmes fils rigides flexibles fixés à ladite extrémité de la gaine de récupération et enroulés suivant un deuxième sens inverse au premier autour de l'axe longitudinal susdit, chaque fil enroulé dans un desdits sens croisant des fils enroulés dans l'autre sens suivant une disposition tressée. De tels tuteurs sont connus en soi (voir GB-1205743, US-A-4655711 et US-A-4954126). Des tuteurs, autres que des tuteurs en fils tressés, peuvent aussi être prévus comme moyens de récupération à fixer sur la gaine de récupération 11, par exemple des tuteurs selon l'enseignement du brevet US-A-4580568. On peut prévoir des tuteurs à fils présentant une élasticité propre ou plutôt une mémoire thermique. Ces tuteurs peuvent enfin être avantageusement recouverts, au moins partiellement, sur leur paroi externe et/ou interne d'un recouvrement en matière synthétique (voir EP-A-0603959), par exemple en polyuréthanne, polycarbonate, silicone, Dacron®, polytétrafluoroéthylène.

On peut aussi prévoir, comme moyens de récupération, un tube flexible formé par exemple de branches flexibles 15 (voir figure 10) qui s'étendent axialement à l'extrémité de la gaine de récupération 11 et qui s'écartent radialement mutuellement quand elles sont à l'extérieur de la gaine externe. Ces branches 15 peuvent être recouvertes, à la manière d'un parapluie, d'un recouvrement 16 en matière synthétique.

On comprendra qu'en fonction du mode de fabrication du moyen de récupération, on pourra obtenir, dans son état évasé, une forme conique, hyperbolique, de voile tendue sur des branches rectilignes ou une forme analogue. Dans la suite on désignera d'une manière générale ce moyen par cône de récupération.

Dans l'exemple de réalisation représenté sur les figures 5 à 7, le poussoir 3 présente à son tour une cavité axiale dans laquelle peut glisser une tige coulissante 18. Des moyens de commande, connus en soi et non représentés, sont prévus pour le déplacement relatif entre le poussoir 3 et la tige coulissante 18. Cette tige 18 supporte à son extrémité distale un organe connu en soi, appelé cône d'introduction 19. Le cône d'introduction 19 présente une extrémité distale qui s'amincit et facilite ainsi l'introduction du dispositif de capture dans les cavités du corps humain ou animal. On peut par exemple prévoir dans cet usage des introducteurs mis sur le marché par la firme BALT Extrusion, France. Ainsi qu'il ressort de la figure 5, le cône d'introduction 19 peut être partiellement logé à l'intérieur de la cavité 2 de la gaine porteuse 1, en position de non-usage.

La tige coulissante 18 doit aussi être creuse et être traversée par un fil de guidage 20 connu en soi, qui est destiné à faciliter le passage de l'introducteur 19 dans la cavité du corps humain ou animal. Dans l'exemple de réalisation illustré le fil de guidage 20 coulisse dans la tige 18 et présente une extrémité épaisse 21 pour provoquer le retrait du cône d'introduction 19 lorsqu'on tire sur le fil 20.

D'une manière connue en soi, le dispositif de capture illustré sur les figures 5 à 7 est introduit dans une cavité du corps humain, par exemple un vaisseau sanguin 23, en faisant passer tout d'abord la tige de guidage 20 à extrémité épaissie 21, puis le cône d'introduction 19 en saillie par rapport à la gaine externe 10, et enfin la gaine externe 10 contenant le reste du dispositif.

Ce dispositif est particulièrement approprié pour la récupération d'un élément tubulaire situé à l'intérieur d'une cavité du corps et qui doit être enlevé totalement de celui-ci. Il peut s'agir par exemple d'une endoprothèse, d'un tuteur, d'un dilatateur ou d'un élément analogue.

Lorsque le dispositif de capture est en face de l'extrémité proximale de l'endoprothèse 22, la gaine porteuse 1 est avancée avec le poussoir 3 jusque devant l'entrée de l'endoprothèse 22 ou légèrement à l'intérieur de celle-ci, puis elle est reculée seule ce qui permet aux éléments d'accrochage 7 de sortir de la gaine porteuse 1, en s'évasant radialement vers l'extérieur. Les crochets 9 peuvent alors s'accrocher dans la paroi interne de l'extrémité proximale de l'endoprothèse 22 (voir figure 6).

La gaine externe est alors tirée vers l'arrière, afin de permettre un évasement vers l'extérieur du tube flexible de récupération en forme de tuteur auto-expansible 14. Celui-ci s'installe comme un cône de récupération ouvert vers l'endoprothèse, prêt à recevoir celle-ci (voir figure 6). Le poussoir 3 est alors enfoncé à nouveau dans la gaine porteuse 1, ce qui rapproche les éléments d'accrochage 7 l'un de l'autre et entraîne un amincissement de l'endoprothèse 22 à récupérer, à son extrémité proximale (voir figure 7). Simultanément l'endoprothèse commence à pénétrer dans le cône de récupération. Quand le poussoir est en bout de course, la gaine porteuse 1 est à son tour tirée vers l'arrière jusqu'à ce que la totalité de l'endoprothèse 22 ait pénétré dans le cône de récupération formé par le tuteur 14. A ce moment, soit la gaine externe 10 est poussée vers l'avant, soit la gaine de récupération 11 est tirée vers l'arrière, soit les deux à la fois, ce qui permet une introduction complète de l'endoprothèse dans le dispositif de capture suivant l'invention et son extraction hors du corps humain ou animal.

On peut à ce moment prévoir de rentrer la base du cône d'introduction 19 à l'intérieur de la gaine porteuse 1, ce qui va enfermer l'endoprothèse à l'intérieur du dispositif de capture.

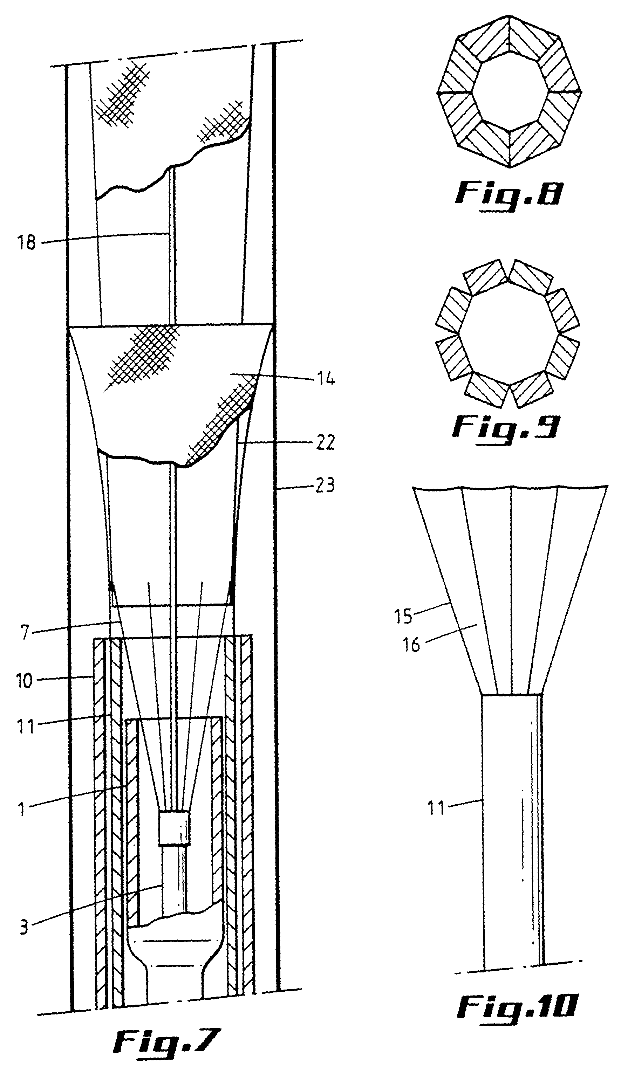

Sur les figures 8 et 9 on a illustré deux variantes de réalisation d'éléments d'accrochage 7 dans une vue en section analogue à la figure 3. Sur la figure 9 ils s'agit de fils à section quadrangulaire et sur la figure 8 de fils à section trapézoïdale. Les formes de réalisation suivant les figures 8 et 9, et en particulier celle selon la figure 8, offrent l'avantage d'empêcher un entremêlement éventuel des fils 7 lors de leur rentrée dans la gaine porteuse 1.

Les différentes gaines utilisées pour la fabrication des formes de réalisation suivant l'invention sont disponibles sur le marché, par exemple chez la firme ADAM Spence Corporation. On peut prévoir des gaines en Teflon®, en polyéthylène, en Nylon®, ou tout autre matière appropriée dans ce but.

Dans cet exemple de réalisation, la gaine externe 10 aura par exemple un diamètre externe de 6-7mm, avec une épaisseur inférieure à 1 mm, la gaine de récupération 11 un diamètre externe de 5 mm, la gaine porteuse 1 un diamètre externe de 4 mm et le poussoir un diamètre externe de 3 mm. L'extrémité proximale du cône d'introduction 19 a un diamètre de 3 mm et la tige coulissante 18 a un diamètre interne inférieur à 1 mm.

Il doit être entendu que la présente invention n'est en aucune façon limitée aux formes de réalisation décrites ci-dessus et qu'on peut envisager diverses modifications dans le cadre de l'invention indiquée dans les revendications ci-après.

On peut par exemple envisager que le cône de récupération 14 soit agencé à l'extrémité de la gaine porteuse 1, ce qui permettait de supprimer la gaine de récupération 11.

## Revendications

1. Dispositif de capture apte à être introduit dans un conduit tubulaire biologique d'un corps humain ou animal, pour capturer un matériel tubulaire situé dans ce conduit, comprenant
une gaine porteuse (1) présentant une première cavité axiale (2),
des moyens de poussoir (3) logés à l'intérieur de la gaine porteuse (1) et capables de coulisser dans ladite première cavité axiale (2),
des premiers moyens de commande (4, 5) permettant un déplacement relatif entre les moyens de poussoir (3) et la gaine porteuse (1), et
des moyens de préhension flexibles (7) qui sont portés par les moyens de poussoir (3) et sont déplacés entre une position à l'extérieur de la gaine porteuse (1), où ils sont écartés l'un de l'autre, et une position à l'intérieur de la gaine porteuse (1), où ils sont rapprochés l'un de l'autre, les moyens de préhension présentant une extrémité distale courbée vers l'extérieur,
**caractérisé en ce que** les moyens de préhension comprennent des éléments d'accrochage (7) qui sont disposés périphériquement sur un poussoir (3) de manière à s'étendre vers l'avant, à l'intérieur de la gaine porteuse (1), et à s'écarter l'un de l'autre radialement à l'extérieur de la gaine porteuse (1), et qui sont chacun pourvus à leur extrémité distale d'un crochet (9) recourbé radialement vers l'extérieur, crochets qui, lorsque les éléments d'accrochage (7) sont situés à l'intérieur du matériel tubulaire et sont amenés dans ladite position à l'extérieur de la gaine porteuse, sont capables d'entrer en position d'accrochage avec une surface interne du matériel tubulaire et qui, lorsque les éléments d'accrochage (7) se trouvent dans la position d'accrochage susdite et sont amenés vers ladite position à l'intérieur de la gaine porteuse (1), permettent de comprimer radialement le matériel tubulaire.

2. Dispositif suivant la revendication 1, **caractérisé en ce que** les éléments d'accrochage sont des fils minces flexibles (7) qui, à une extrémité, sont fixés à une extrémité distale du poussoir (3) et sont élastiquement comprimés par la gaine porteuse (1) lorsqu'ils sont à l'intérieur de celle-ci.

3. Dispositif suivant l'une ou l'autre des revendications 1 et 2, **caractérisé en ce qu'**il comprend au moins 3, avantageusement au moins 6, de préférence au moins 8, éléments d'accrochage (7).

4. Dispositif suivant l'une quelconque des revendications 1 à 3, **caractérisé en ce qu'**il comprend en outre
une gaine externe (10) présentant une deuxième cavité axiale (12), dans laquelle la gaine porteuse (1) est logée de manière à pouvoir coulisser,
des deuxièmes moyens de commande permettant un déplacement relatif entre la gaine externe (10) et la gaine porteuse (1),
et des moyens de récupération de l'objet capturé par les éléments d'accrochage (7), qui sont déplaçables entre une position à l'extérieur de la gaine externe (10) et une position à l'intérieur de la gaine externe (10).

5. Dispositif suivant la revendication 4, **caractérisé en ce que** les moyens de récupération sont portés à une extrémité de la gaine porteuse (1).

6. Dispositif suivant la revendication 4, **caractérisé en ce qu'**il comprend en outre
une gaine de récupération (11) qui présente une troisième cavité axiale (13), dans laquelle la gaine porteuse (1) est logée de manière à pouvoir coulisser, et qui est elle-même logée dans ladite deuxième cavité axiale (12) de la gaine externe (10) de manière à pouvoir coulisser dans celle-ci,
**en ce que** les deuxièmes moyens de commande comprennent des moyens de déplacement relatif entre la gaine externe (10) et la gaine de récupération (11), et des moyens de déplacement relatif entre la gaine de récupération (11) et la gaine porteuse (1), et
**en ce que** les moyens de récupération sont portés à une extrémité de la gaine de récupération (11).

7. Dispositif suivant l'une quelconque des revendications 4 à 6, **caractérisé en ce que** les moyens de récupération sont constitués d'un tube flexible (14-16), qui est comprimé à l'intérieur de la deuxième cavité axiale (12) de la gaine externe (10) et qui, à l'extérieur de celle-ci, s'évase en étant ouvert vers le conduit tubulaire biologique du corps humain ou animal.

8. Dispositif suivant la revendication 7, **caractérisé en ce que** le tube flexible (14) formant les moyens de récupération est un tuteur auto-expansible qui est radialement expansible et axialement rétractable et qui comprend des premiers fils rigides flexibles fixés à une extrémité de la gaine de récupération (11) ou respectivement de la gaine porteuse (1) et enroulés suivant un premier sens autour d'un axe longitudinal de celle-ci et des deuxièmes fils rigides flexibles fixés à ladite extrémité de la gaine de récupération (11) ou respectivement de la gaine porteuse (1) et enroulés suivant un deuxième sens inverse au premier autour de l'axe longitudinal susdit, chaque fil enroulé dans un desdits sens croisant des fils enroulés dans l'autre sens suivant une disposition tressée.

9. Dispositif suivant la revendication 7, **caractérisé en ce que** le tube flexible (15, 16) formant les moyens de récupération est constitué de branches flexibles (15) qui s'étendent axialement à l'extrémité de la gaine de récupération (11) ou repectivement de la gaine porteuse (1), et qui s'écartent radialement mutuellement à l'extérieur du dispositif.

10. Dispositif suivant l'une des revendications 7 à 9, **caractérisé en ce que** le tube flexible (14-16) présente un recouvrement interne et/ou externe, au moins partiel, en une matière appropriée.

11. Dispositif suivant l'une quelconque des revendications 1 à 10, **caractérisé en ce que** le poussoir (3) comprend une quatrième cavité axiale, et **en ce que** le dispositif comprend en outre
une tige coulissante (18) logée à l'intérieur de la quatrième cavité axiale et capable de coulisser dans celle-ci,
des troisièmes moyens de commande permettant un déplacement relatif entre la tige coulissante (18) et le poussoir (18), et
un élément d'introduction (19) porté par la tige coulissante (18), pour faciliter l'introduction du dispositif de capture dans le conduit tubulaire biologique du corps humain ou animal.

12. Dispositif suivant la revendication 11, **caractérisé en ce que** la gaine porteuse (1) comporte à une extrémité distale un logement dans lequel l'élément d'introduction (19) peut au moins partiellement être logé.

13. Dispositif suivant l'une des revendications 11 et 12, **caractérisé en ce que** la tige coulissante (18) présente une cinquième cavité axiale et **en ce que** le dispositif comprend en outre un fil de guidage (20) éventuellement pourvu d'un épaississement (21) à une extrémité distale, qui est logé à l'intérieur de la cinquième cavité axiale de manière à pouvoir coulisser dans celle-ci, et à faciliter le passage de l'élément d'introduction (19) dans le conduit tubulaire biologique du corps humain ou animal.

## Claims

1. Capturing device able to be introduced into a tubular biological conduit of a human or animal body, in order to capture a tubular article situated in this conduit, comprising
a support sheath (1) having a first axial cavity (2),
pusher means (3) accommodated inside the support sheath (1) and capable of sliding in the said first axial cavity (2),
first control means (4, 5) permitting a relative displacement between the pusher means (3)and the support sheath (1), and
flexible gripping means (7) which are borne by the pusher means (3) and are displaced between a position outside the support sheath (1), in which position they are spread apart from one another, and a position inside the support sheath (1), in which position they are drawn together, the gripping means having a distal end curved outwards,
**characterized in that** the gripping means comprise fastening elements (7) which are disposed peripherally on a pusher (3) in such a way as to extend forwards, inside the support sheath (1), and to spread apart radially from one another outside the support sheath (1), and which fastening elements (7) are each provided at their distal end with a hook (9) which is curved radially outwards, which hooks, when the fastening elements (7) are situated inside the tubular article and are brought into the said position outside the support sheath, are capable of entering into a position of fastening to an inner surface of the tubular article, and which, when the fastening elements (7) are situated in the abovementioned fastening position and are brought towards the said position inside the support sheath (1), allow to radially compress the tubular article.

2. Device according to claim 1, **characterized in that** the fastening elements are thin flexible wires (7) which, at one end, are fixed to a distal end of the pusher (3) and are elastically compressed by the support sheath (1) when they are inside the latter.

3. Device according to one or other of claims 1 and 2, **characterized in that** it comprises at least 3, advantageously at least 6, preferably at least 8, fastening elements (7).

4. Device according to any one of claims 1 to 3, **characterized in that** it additionally comprises
an external sheath (10) having a second axial cavity (12) in which the support sheath (1) is accommodated in such a way as to be able to slide,
second control means permitting a relative displacement between the external sheath (10) and the support sheath (1),
and means for recovering the article captured by the fastening elements (7), which means are displaceable between a position outside the external sheath (10) and a position inside the external sheath (10).

5. Device according to claim 4, **characterized in that** the recovery means are borne at one end of the support sheath (1).

6. Device according to claim 4, **characterized in that** it additionally comprises
a recovery sheath (11) which has a third axial cavity (13) in which the support sheath (1) is accommodated in such a way as to be able to slide, and which is itself accommodated in the said second axial cavity (12) of the external sheath (10) in such a way as to be able to slide in the latter,
**in that** the the second control means comprising means for relative displacement between the external sheath (10) and the recovery sheath (11), and means for relative displacement between the recovery sheath (11) and the support sheath (1), and
**in that** the recovery means being borne at one end of the recovery sheath (11).

7. Device according to any one of claims 4 to 6, **characterized in that** the recovery means are made up of a flexible tube (14-16) which is compressed inside the second axial cavity (12) of the external sheath (10) and which, outside the latter, widens out, being open towards the tubular biological conduit of the human or animal body.

8. Device according to claim 7, **characterized in that** the flexible tube (14) forming the recovery means is a self-expandable stent which is radially expandable and axially retractable and which comprises first flexible, rigid wires which are fixed at one end of the recovery sheath (11) or, respectively, of the support sheath (1) and are wound in a first direction about a longitudinal axis thereof, and second flexible, rigid wires which are fixed at the said end of the recovery sheath (11) or, respectively, of the support sheath (1) and are wound in a second direction, counter to the first direction, about the abovementioned longitudinal axis, each wire wound in one of the said directions crossing wires wound in the other direction to give a braided arrangement.

9. Device according to claim 7, **characterized in that** the flexible tube (15, 16) forming the recovery means is made up of flexible branches (15) which extend axially at the end of the recovery sheath (11) or, respectively, of the support sheath (1), and which spread apart radially from each other outside the device.

10. Device according to one of claims 7 to 9, **characterized in that** the flexible tube (14-16) has an at least partial internal and/or external covering made of a suitable material.

11. Device according to any one of claims 1 to 10, **characterized in that** the pusher (3) comprises a fourth axial cavity, and the device additionally comprises
a sliding rod (18) accommodated inside the fourth axial cavity and capable of sliding in the latter,
third control means permitting a relative displacement between the sliding rod (18) and the pusher (3), and
an introducer element (19) borne by the sliding rod (18), in order to facilitate the introduction of the capturing device into the tubular biological conduit of the human or animal body.

12. Device according to claim 11, **characterized** the support sheath (1) includes, at a distal end, a seat in which the introducer element (19) can at least partially be accommodated.

13. Device according to any one of claims 11 and 12, **characterized in that** the sliding rod (18) has a fifth axial cavity, and **in that** the device additionally comprises a guide wire (20) optionally provided with a thickened portion (21) at a distal end, which is accommodated inside the fifth axial cavity in such a way as to be able to slide in the latter, and to facilitate the passage of the introducer element (19) in the tubular biological conduit of the human or animal body.

## Patentansprüche

1. Fangvorrichtung, welche in einen biologischen Leitungskanal eines menschlichen oder tierischen Körpers einführbar ist, um ein rohrförmiges Material einzufangen, welches sich in dem Kanal befindet, welche folgendes aufweist:
einen Tragmantel (1), welcher einen ersten axialen Hohlraum (2) hat,
eine Schiebeeinrichtung (3), welche am Innern des Tragmantels (1) angeordnet ist und in dem ersten axialen Hohlraum (2) eine Gleitbewegung ausführen kann,
eine erste Betätigungseinrichtung (4, 5), welche eine relative Verschiebung zwischen der Schiebeeinrichtung (3) und dem Tragmantel (1) gestattet, und
eine flexible Greifeinrichtung (7), die von der Verschiebeelnrichtung (3) getragen wird und zwischen einer Position außerhalb des Tragmantels (1), in welcher die Greifeinrichtung beabstandet ist, und einer Position im Innern des Tragmantels (1) verschlebbar ist, zu welchem die Greifeinrichtung benachbart Ist, wobei die Greifeinrichtung ein in Richtung nach außen gebogenes distales Ende hat,
**dadurch gekennzeichnet, daß** die Greifeinrichtung Hängeelemente (7) aufweist, welche in Umfangsrichtung auf einem Schiebestück (3) nach vorne weisend im Innern des Tragmantels (1) angeordnet sind, die zueinander radial außerhalb des Tragmantels (1) einen Abstand haben, und die jeweils mit einem Haken (9) mit einem distalen Ende versehen sind, welcher nach außen gekrümmt verläuft wobei die Haken (9), wenn die Hängeelemente im Innern des rohrförmigen Kanals angeordnet sind und sich in der Position außerhalb des Tragmantels befinden, In eine Hängeposition mit der Innenfläche des rohrförmigen Materials eintreten können, wenn die in der in der Hängeposition befindlichen Hängeelementen (7) eine Position im Innern des Tragmantels (1) radial zu dem rohrförmigen Material einnehmen.

2. Vorrichtung nach Anspruch 1, **dadurch gekennzeichnet, daß** die Hängeelemente dünne, flexible Drähte (7) sind, die an einem Ende an einem distalen Ende des Druckstücks (3) befestigt sind und sich elastisch durch den Tragmantel (1) zusammendrücken lassen, wenn sie sich im Innern desselben befinden.

3. Vorrichtung nach Anspruch 1 oder 2, **dadurch gekennzeichnet, daß** sie wenigstens drei, vorzugsweise sechs, und insbesondere acht Hängeelemente (7) aufweist.

4. Vorrichtung nach einem der Ansprüche 1 bis 3, **dadurch gekennzeichnet, daß** sie ferner folgendes aufweist:
einen äußeren Mantel (10), welcher einen zweiten axialen Hohlraum (12) hat, In dem der Tragmantel (1) gleitbeweglich angeordnet ist,
eine zweite Betätigungseinrichtung eine relative Verschiebung zwischen dem äußeren Mantel (10) und dem Tragmantel (1) gestattet, und
die Rückgewinnungseinrichtung den Gegenstand mittels der Hängeelemente (7) einfängt, welche zwischen einer Position außerhalb des äußeren Mantels (10) und einer Position Im Innern des äußeren Mantels (10) verschiebbar sind.

5. Vorrichtung nach Anspruch 4, **dadurch gekennzeichnet, daß** die Rückgewinnungseinrichtung an einem Ende des Tragmantels (1) vorgesehen ist.

6. Vorrichtung nach Anspruch 4, **dadurch gekennzeichnet, daß** sie ferner folgendes aufweist:
einen Rückgewinnungsmantel (11), welcher einen dritten axialen Hohlraum (13) aufweist, in welchem der Tragmantel (1) gleitbeweglich angeordnet ist, und der Rückgewinnungsmantel selbst in dem zweiten axialen Hohlraum (12) des äußeren Mantels (10) gleitbeweglich in demselben angeordnet ist,
die zweite Betätigungseinrichtung eine Einrichtung für eine relative Verschiebung zwischen dem äußeren Mantel (10) und dem Rückgewinnungsmantel (11) und zur relativen Verschiebung zwischen dem Rückgewinnungsmantel (11) und dem Tragmantel (1) umfaßt, und
die Rückgewinnungseinrichtung an einem Ende des Rückgewinnungsmantels (11) angebracht ist.

7. Vorrichtung nach einem der Ansprüche 4 bis 6, **dadurch gekennzeichnet, daß** die Rückgewinnungseinrichtung von einem flexiblen Rohr (14-16) gebildet wird, welches im Innern des zwelten axialen Hohlraums (12) des äußeren Mantels (10) zusammengedrückt ist, und die von diesem nach außen unter Bildung einer Öffnung in Richtung des biologischen Leitungskanals des menschlichen oder tierischen Körpers vorstehen kann.

8. Vorrichtung nach Anspruch 7, **dadurch gekennzeichnet, daß** das flexible Rohr (14), welches die Rückgewinnungseinrichtung bildet, eine selbstexpandierbare Stütze ist, die sich in radialer Richtung aufweiten und in axlaler Richtung zusammenziehen kann, und die erste starre Drähte aufweist, die flexibel an einem Ende des Rückgewinnungsmantels (11) oder an dem Tragmantel (1) angebracht sind, und die in einer ersten Richtung um eine Längsachse desselben aufgewickelt werden können, und zweite starre Drähte aufweist, welche flexibel an dem Ende des Rückgewinnungsmantels (11) oder an dem Tragmantel (1) angebracht sind, und in einer zweiten Richtung umgekehrt zur ersten Richtung um diese Längsachse aufgewickelt werden können, wobei jeder in der einen Richtung aufgewickelte Draht in der anderen Richtung aufgewickelte Drähte an versetzten Stellen kreuzt.

9. Vorrichtung nach Anspruch 7, **dadurch gekennzeichnet, daß** das flexible Rohr (15, 16), welches die Rückgewinnungseinrichtung bildet, von flexiblen Zweigabschnitten (15) gebildet werden, welche sich axial von einem Ende des Rückgewinnungsmantels (11) oder des Tragmantels (1) erstrecken, und die wechselseitig radial zu der Außenseite der Vorrichtung einen Abstand haben.

10. Vorrichtung nach einem der Ansprüche 7 bis 9, **dadurch gekennzeichnet, daß** das flexible Rohr (14-16) eine innere und/oder äußere Überzugsschicht aus einem geeigneten Material aufweist.

11. Vorrichtung nach einem der Ansprüche 1 bis 10, **dadurch gekennzeichnet, daß** das Druckstück (3) einen vierten axialen Hohlraum aufweist, und daß die Vorrichtung ferner folgendes aufweist:
eine Gleitstange (18), welche im Innern des vierten axialen Hohlraums gleitbeweglich angeordnet ist,
eine dritte Betätigungseinrichtung für eine relative Verschiebung zwischen der Gleitstange (18) und der Schiebeeinrichtung (18), und
ein Einführungselement (19), welches an der Gleitstange (18) angebracht ist, um das Einführen der Fangvorrichtung in den biologischen Leitungskanal des menschlichen oder tierischen Körpers zu erleichtern.

12. Vorrichtung nach Anspruch 11, **dadurch gekennzeichnet, daß** der Tragmantel (1) an einem distalen Ende eine Aufnahme aufweist, in welcher das Einführungselement (19) wenigstens teilweise untergebracht ist.

13. Vorrichtung nach Anspruch 11 oder 12, **dadurch gekennzeichnet, daß** die Gleitstange (18) einen fünften axialen Hohlraum aufweist, und daß die Vorrichtung ferner einen Führungsdraht (20) aufweist, welcher gegebenenfalls mit einer Verdickung (21) am distalen Ende versehen ist, und der im Inneren des fünften axialen Hohlraums gleitbeweglich in demselben angeordnet ist, und zur Erleichterung des Durchgangs des Einführungselements (19) in den biologischen Leitungskanal des menschlichen oder tierischen Körpers dient.
